(19) Europäisches Patentamt
European Patent Office
Office européen des brevets

(11) Veröffentlichungsnummer: **0 138 181**
**A2**

(12) **EUROPÄISCHE PATENTANMELDUNG**

(21) Anmeldenummer: **84112031.4**

(22) Anmeldetag: **08.10.84**

(51) Int. Cl.[4]: **C 07 D 307/86**, A 01 N 47/22

(30) Priorität: **18.10.83 DE 3337858**

(43) Veröffentlichungstag der Anmeldung: **24.04.85 Patentblatt 85/17**

(84) Benannte Vertragsstaaten: **BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Heywang, Gerhard, Dr., Nittumer Weg 4, D-5060 Bergisch-Gladbach 2 (DE)**
Erfinder: **Kühle, Engelbert, Dr., Von-Bodelschwingh-Strasse 42, D-5060 Bergisch-Gladbach 2 (DE)**
Erfinder: **Homeyer, Bernhard, Dr., Obere Strasse 28, D-5090 Leverkusen 3 (DE)**

(54) **N-Oxalylderivate von N-Methylcarbamaten, Verfahren zu ihrer Herstellung und ihre Verwendung als Schädlingsbekämpfungsmittel.**

(57) Die vorliegende Erfindung betrifft neue N-Oxalyl-N-methyl-carbamidsäureester der Formel I

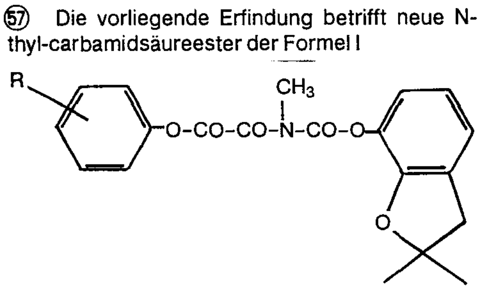

(I)

in welcher
R für Alkyl, Alkenyl, Alkinyl, Alkoxy, Alkylthio, Halogenalkyl, Halogenalkoxy, Halogen-alkylthio, Halogen, Nitro, Dialkylamino, Alkylsulfinyl, Alkylsulfonyl, Cycloalkyl oder gesättigte, gegebenenfalls substituierte, Heterocyclen mit einem oder mehreren Heteroatomen aus der Gruppe O, S, N steht, oder einen an den Phenylrest anellierten Ring bildet, der gegebenenfalls ein oder mehrere Heteroatome aus der Gruppe O, S, N enthält und gegebenenfalls substituiert ist.

Man erhält sie, indem man N-Chloroxalyl-N-methyl-2,3-dihydro-2,2-dimethyl-benzofuran-7-yl-carbamidsäureester mit Phenolen gegebenenfalls in Gegenwart eines Verdünnungsmittels und/oder gegebenenfalls in Gegenwart einer Base umsetzt.

Die erfindungsgemäßen Verbindungen zeichnen sich durch gute Wirksamkeit als Schädlingsbekämpfungsmittel, insbesondere Insektizide, Akarizide und Nematozide aus.

EP 0 138 181 A2

BAYER AKTIENGESELLSCHAFT 5090 Leverkusen, Bayerwerk

Konzernverwaltung RP
Patentabteilung Rt/ABc

Ia

N-Oxalylderivate von N-Methylcarbamaten, Verfahren zu ihrer Herstellung und ihre Verwendung als Schädlingsbekämpfungsmittel

Die vorliegende Erfindung betrifft N-Oxalylderivate von Methylcarbamaten, Verfahren zu ihrer Herstellung und ihre Verwendung als Schädlingsbekämpfungsmittel.

Es ist bereits bekannt geworden, daß N-carboxylierte N-Methylcarbamidsäurearylester (vgl. DE-OS 2 132 936) und N-Chlorcarbonyl-N-methylcarbamidsäurearylester (vgl. DE-OS 2 142 496) sowie N-Oxalyl-N-methyl-carbamidsäurearylester (vgl. DE-OS 3 205 195) insektizide Eigenschaften haben. Ihre Wirkung ist jedoch, vor allem bei niedrigen Aufwandmengen, nicht immer voll befriedigend.

Es wurden nun neue N-Oxalyl-N-methyl-carbamidsäureester der Formel I gefunden

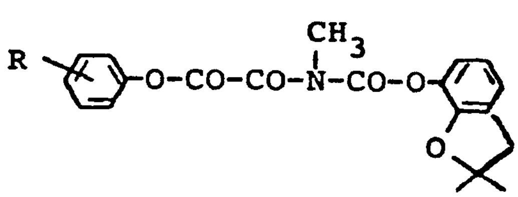

(I)

in welcher

R für Alkyl, Alkenyl, Alkinyl, Alkoxy, Alkylthio, Halogenalkyl, Halogenalkoxy, Halogen-alkylthio, Halogen, Nitro, Dialkylamino, Alkylsulfinyl, Alkylsulfonyl, Cycloalkyl oder gesättigte, gegebenenfalls substituierte, Heterocyclen mit einem oder mehreren Heteroatomen aus der Gruppe O, S, N steht, oder einen an den Phenylrest anellierten Ring bildet, der gegebenenfalls ein oder mehrere Heteroatome aus der Gruppe O, S, N enthält und gegebenenfalls substituiert ist.

Weiterhin wurde gefunden, daß man die neuen Carbamidsäureester der Formel I erhält, indem man N-Chloroxalyl-N-methyl-2,3-dihydro-2,2-dimethyl-benzofuran-7-yl-carbamidsäureester der Formel II

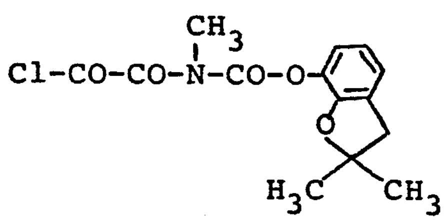

(II)

mit Phenolen der allgemeinen Formel (III)

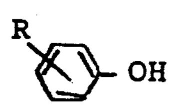

(III)

in welcher

R die oben angegebene Bedeutung hat,

gegebenenfalls in Gegenwart eines Verdünnungsmittels und/oder gegebenenfalls in Gegenwart einer Base umsetzt.

Die erfindungsgemäßen Verbindungen zeichnen sich durch gute Wirksamkeit als Schädlingsbekämpfungsmittel, insbesondere als Insektizide, Akarizide und Nematozide aus.

Es ist ausgesprochen überraschend, daß sie bei günstiger Warmblütertoxizität eine höhere Wirkung zeigen als die aus dem Stand der Technik bekannten Oxalyl-N-methylcarbamate.

Die erfindungsgemäßen Verbindungen sind durch die Formel I allgemein definiert; darin steht

R bevorzugt für $C_1$-$C_4$-Alkyl, $C_2$-$C_4$-Alkenyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio mit jeweils bis zu 5 Halogenatomen, Fluor, Chlor, Brom, Nitro, Di($C_1$-$C_4$-alkyl)-amino, $C_1$-$C_4$-Alkylsulfinyl, $C_1$-$C_4$-Alkylsulfonyl, $C_3H_8$-Cycloalkyl, Tetrahydrofuranyl, Dioxolanyl, Pyrrolidinyl, Piperidinyl, Morpholinyl, N-Methyl-piperazinyl, die gegebenenfalls durch $C_1$-$C_4$-Alkyl oder Halogen substituiert sind

R bildet ferner vorzugsweise einen an zwei benachbarten C-Atomen des Phenylrings anellierten, gesättig-5- oder 6-Ring, der gegebenenfalls Heteroatome wie O, S oder N enthalten kann und gegebenenfalls durch Halogen oder $C_1$-$C_4$-Alkyl substituiert ist, insbesondere seien genannt:
$O-CH_2-O-$; $-O-CF_2-O-$; $O-CH_2-CF_2-O-$; $-O-CF_2-CF_2-O-$; $-O-CF_2-O-CF_2-$; $-CH_2-C(CH_3)_2-O-$, $-O-CH(CH_3)-O-$, $-O-C(CH_3)_2-O-$, $-O-CH(CH_3)-O-$, $-O-C(CH_3)_2-O-$, $-CH_2-C(CH_3)_2-CH_2$; $-O-CH_2-C(CH_3)_2-O$, $-CH_2-O-C(CH_3)_2-O-$.

Besonders bevorzugt sind Verbindungen der Formel I, in welcher

R für Methyl, Ethyl, Propyl, i-Propyl, Butyl, i-Butyl, s-Butyl, t-Butyl, Allyl, Methoxy, Ethoxy, Propoxy, i-Propoxy, Butyloxy, i-Butyloxy, s-Butyloxy, t-Butyloxy, Methylthio, Chlormethyl, Trichlormethyl, Trifluormethyl, Trifluormethoxy, Trifluormethylthio, Fluor, Chlor, Brom, Nitro, Dimethylamino, Diethylamino, Methylsulfinyl, Methylsulfonyl, Cyclopentyl, Cyclohexyl, Tetrahydrofuranyl, Dioxolanyl, Dioxanyl, Pyrrolidinyl, Piperidinyl, Morpholinyl steht, oder einen an zwei benachbarten C-Atomen des Phenylrest anellierten Ring bildet, wobei R für folgende Reste steht:
$-O-CH_2-O-$; $-CH_2-C(CH_3)_2-O-$,$-O-CH(CH_3)-O-$, $-O-C(CH_3)_2-O-$, $-CH_2-C(CH_3)_2-CH_2-$.

Ganz besonders bevorzugt sind die folgenden Verbindungen:

2-[N-7-(2,3-Dihydro-2,2-dimethylbenzofuranyl)-oxycarbonyl-N-methyl]-amino-2-oxo-ethansäure--methylphenylester
-i-propylphenylester
-methoxyphenylester
-i-propoxyphenylester
-trifluormethoxyphenylester
-chlorphenylester
-nitrophenylester
-2,3-dioxomethylenphenylester
-2,3-dioxo-isopropylidenphenylester
-7(2,3-dihydro-2,2-dimethylbenzofuranyl)ester.

Verwendet man 2,3-Dihydro-2,2-dimethyl-7-benzofuranyl-N-chloroxalyl-N-methylcarbamat (Formel IV) und 4-Chlorphenol (Formel V) als Ausgangsstoffe, so läßt sich der Reaktionsverlauf durch das folgende Formelschema wiedergeben:

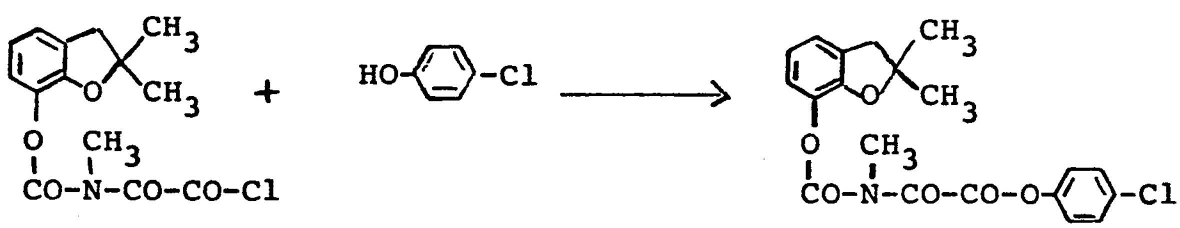

IV V

Als Verdünnungsmittel für die Herstellung der erfindungsgemäßen Verbindungen eignen sich inerte organische Lösungsmittel. Hierzu gehören Ether wie Diethylether, Dioxan, Tetrahydrofuran, Kohlenwasserstoffe wie Benzol oder Toluol, chlorierte Kohlenwasserstoffe wie Methylenchlorid, Chloroform oder Chlorbenzole, weiterhin Nitrile, Ketone, Ester sowie Gemische aus diesen Lösungsmitteln.

Als säurebindende Mittel setzt man dem Reaktionsgemisch bevorzugt Basen wie z.B. Natriumcarbonat, Natriumhydrogencarbonat oder tertiäre organische Basen, wie z.B. Triethylamin oder Benzyldimethylamin zu.

Die Reaktionstemperatur kann in einem größeren Bereich variiert werden. Im allgemeinen arbeitet man zwischen 0 und 100°C.

Gewöhnlich werden die Reaktionspartner in äquimolaren Mengen eingesetzt, doch auch die Verwendung einer Komponente im Überschuß ist möglich.

Die Herstellung des N-Chloroxalyl-carbamidsäureesters der Formel (II) ist bekannt (vgl. DE-OS 3 205 195). Die benötigten Phenole der Formel (III) sind bekannt oder können nach bekannten Methoden hergestellt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additve können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung

mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen),

Aufgießens (pour-on and spot-on) und des Einpuderns sowie durch parenterale Anwendung in Form beispielsweise der Injektion.

Herstellungsbeispiele

Herstellung der Ausgangsverbindung 2,3-Dihydro-2,2-dimethyl-benzofuranyl-7-N-chloroxalyl-N-methyl-carbamat

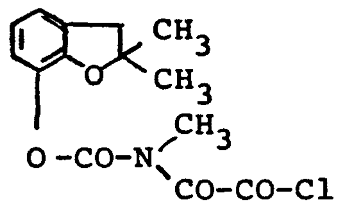

Zu 12,2 g 2,3-Dihydro-2,2-dimethyl-benzofuranyl-7-N-methyl-carbamat in 100 ml Toluol werden 5,4 ml Oxalylchlorid zugetropft und anschließend wird das Gemisch langsam auf 60 - 80°C erwärmt bis zum Ende der Gasentwicklung (etwa 4 Stunden). Die so entstandene Lösung kann für die Umsetzung zu den erfindungsgemäßen Verbindungen benutzt werden. Nach Abdestillieren des Toluols im Vakuum bleiben 15 g zähflüssiges Öl zurück, das nach Verreiben mit Diisopropylether kristallisiert. Fp. 91-93°C, 14,7 g ≙ 96 % d.Th.

1. 2-/N-7-(2,3-Dihydro-2,2-dimethylbenzofuranyl)-oxycarbonyl-N-methyl/-amino-2-oxo-ethansäure-4-chlorphenylester

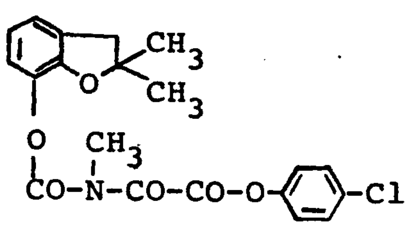

Zu 31,1 g 2,3-Dihydro-2,2-dimethylbenzofuranyl-7-N-oxalyl-N-methyl-carbamat in 400 ml Toluol werden bei Raumtemperatur langsam 12,8 g 4-Chlorphenol eingetragen und anschließend 13,7 g Triethylamin zugetropft. Nach vier Stunden wird die Toluolphase abgetrennt, getrocknet, eingeengt und der Rückstand aus Ethanol umkristallisiert. 23 g (57 % d.Th.), Fp. 148-150°C.

Auf entsprechende Weise wurden die folgenden Oxalylderivate hergestellt:

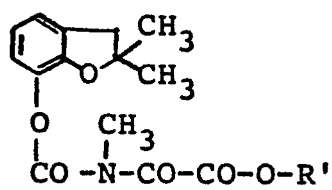

| Typ Nr. | R' | Ausbeute (%) | Fp. (°C) |
|---|---|---|---|
| 2 | 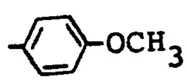 | 93 | 152-154 |
| 3 | 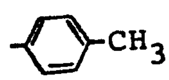 | 94 | 116-118 |
| 4 | 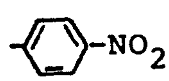 | 86 | 120-125 |
| 5 | 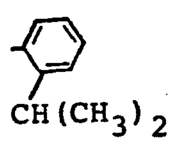 | 47 | 89-90 |

| Typ Nr. | R' | Ausbeute | Fp. (°C) |
|---|---|---|---|
| 6 | 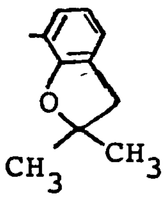 | 83 | 123-126 |
| 7 | 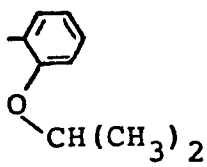 | 80 | 125-127 |

Beispiel A

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 3, 4, 5, 6.

Beispiel B

Wirkungsdauer-Test / Bodeninsekten

Testinsekt: Phorbia antiqua-Maden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (mg/l) angegeben wird. Man füllt den Boden in 5 l Töpfe und läßt diese bei 20°C stehen.

Im Abstand von 2 Wochen werden nach vorheriger erneuter Durchmischung Bodenproben von 250 ccm entnommen und in den behandelten Boden die entsprechenden Testinsekten gesetzt. Nach weiteren 2 bis 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie in der unbehandelten Kontrolle.

Bei diesem Test zeigen z.B. die folgenden Verbindungen der Herstellungsbeispiele überlegene Wirkung gegenüber dem Stand der Technik: 1, 3, 5, 6.

Patentansprüche

1. N-Oxalyl-N-methyl-carbamidsäureester der Formel I

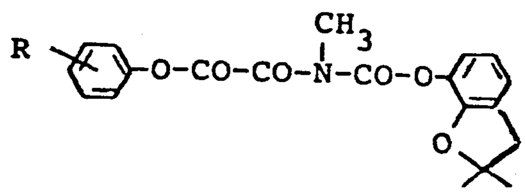

(I)

in welcher

R für Alkyl, Alkenyl, Alkinyl, Alkoxy, Alkylthio, Halogenalkyl, Halogenalkoxy, Halogen-alkylthio, Halogen, Nitro, Dialkylamino, Alkylsulfinyl, Alkylsulfonyl, Cycloalkyl oder gesättigte, gegebenenfalls substituierte, Heterocyclen mit einem oder mehreren Heteroatomen aus der Gruppe O, S, N steht, oder einen an dem Phenylrest anellierten Ring bildet, der gegebenenfalls ein oder mehrere Heteroatome aus der Gruppe O, S, N enthält und gegebenenfalls substituiert ist.

2. Verfahren zur Herstellung der N-Oxalyl-N-methyl-carbamidsäureester der Formel I

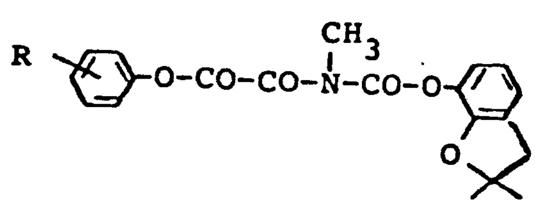

(I)

in welcher

R für Alkyl, Alkenyl, Alkinyl, Alkoxy, Alkylthio, Halogenalkyl, Halogenalkoxy, Halogen-alkylthio, Halogen, Nitro, Dialkylamino, Alkylsulfinyl, Alkylsulfonyl, Cycloalkyl oder gesättigte, gegebenenfalls substituierte, Heterocyclen mit einem oder mehreren Heteroatomen aus der Gruppe O, S, N steht, oder einem an dem Phenylrest anellierten Ring bildet, der gegebenenfalls ein oder mehrere Heteroatome aus der Gruppe O, S, N enthält und gegebenenfalls substituiert ist,

dadurch gekennzeichnet, daß man N-Chloroxalyl-N-methyl-2,3-dihydro-2,2-dimethyl-benzofuran-7-yl-carbamidsäureester der Formel II

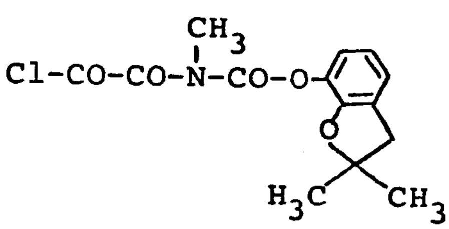

(II)

mit Phenolen der allgemeinen Formel III

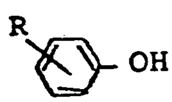

(III)

in welcher

R die oben angegebene Bedeutung hat,

gegebenenfalls in Gegenwart eines Verdünnungsmittels und/oder gegebenenfalls in Gegenwart einer Base umsetzt.

3. Verbindungen der Formel I gemäß Anspruch 1, dadurch gekennzeichnet, daß

R für $C_1$-$C_4$-Alkyl, $C_2$-$C_4$-Alkenyl, $C_1$-$C_4$-Alkoxy, $C_1$-$C_4$-Alkylthio, $C_1$-$C_4$-Halogenalkyl, $C_1$-$C_4$-Halogenalkoxy, $C_1$-$C_4$-Halogenalkylthio mit jeweils bis zu 5 Halogenatomen, Fluor, Chlor, Brom, Nitro, Di($C_1$-$C_4$-Alkyl)-amino, $C_1$-$C_4$-Alkylsulfinyl, $C_1$-$C_4$-Alkylsulfonyl, $C_3$-$C_8$-Cycloalkyl, Tetrahydrofuranyl, Dioxolanyl, Pyrrolidinyl, Piperidinyl, Morpholinyl, N-Methyl-piperazinyl, die gegebenenfalls durch $C_1$-$C_4$-Alkyl oder Halogen substituiert sind, steht, oder für einen an zwei benachbarte C-Atome des Phenylrings anellierten, gesättigten 5- oder 6-Ring steht, der gegebenenfalls Heteroatome wie O, S oder N enthalten kann und gegebenenfalls durch Halogen oder $C_1$-$C_4$-Alkyl substituiert ist.

4. Verbindungen der Formel I gemäß Anspruch 1, dadurch gekennzeichnet, daß

R für Methyl, Ethyl, Propyl, i-Propyl, Butyl, i-Butyl, S-Butyl, t-Butyl, Allyl, Methoxy,

Ethoxy, Propoxy, i-Propoxy, Butyloxy, i-Butyloxy, s-Butyloxy, t-Butyloxy, Methylthio, Chlormethyl, Trichlormethyl, Trifluormethyl, Trifluormethoxy, Trifluormethylthio, Fluor, Chlor, Brom, Nitro, Dimethylamino, Diethylamino, Methylsulfinyl, Methylsulfonyl, Cyclopentyl, Cyclohexyl, Tetrahydrofuranyl, Dioxolanyl, Dioxanyl, Pyrrolidinyl, Piperidinyl, Morpholinyl steht, oder für einen mit zwei benachbarten C-Atomen des Phenylrestes anellierten Ring aus den Resten $-O-CH_2-O-$; $-CH_2-C(CH_3)_2-O-$, $-O-CH(CH_3)-O-$, $-OC(CH_3)_2-O-$, $-CH_2-C(CH_3)_2-CH_2-$ steht.

5. Schädlingsbekämpfungsmittel, gekennzeichnet durch einen Gehalt an mindestens einem Oxalylderivat eines N-Methylcarbamats der Formel (I), gemäß Anspruch 1.

6. Verwendung von Oxalylderivaten von N-Methylcarbamaten der Formel (I) gemäß Anspruch 1 zur Bekämpfung von Schädlingen.

7. Verfahren zur Bekämpfung von Schädlingen, dadurch gekennzeichnet, daß man Oxalylderivate von N-Methylcarbamaten der Formel (I) gemäß Anspruch 1 auf Schädlinge und/oder ihren Lebensraum einwirken läßt.

8. Verfahren zur Herstellung von Schädlingsbekämpfungsmitteln, dadurch gekennzeichnet, daß man Oxalylderivate von N-Methylcarbamaten der Formel (I) gemäß Anspruch 1 mit Streckmitteln und/oder oberflächenaktiven Mitteln vermischt.